Europäisches Patentamt

European Patent Office

Office européen des brevets

(19)

(11) Publication number: 0 142 036

A1

(12) EUROPEAN PATENT APPLICATION

(21) Application number: 84112250.0

(22) Date of filing: 11.10.84

(51) Int. Cl.⁴: **A 61 K 35/78**

(30) Priority: 14.11.83 JP 213686/83

(43) Date of publication of application:
22.05.85 Bulletin 85/21

(84) Designated Contracting States:
BE DE FR GB NL SE

(71) Applicant: THE GREEN CROSS CORPORATION
15-1, Imabashi-1-chome Higashi-ku Osaka-shi
Osaka 541(JP)

(72) Inventor: Iga, Yoshiro
9-33, Danjocho-1-chome
Nishinomiya-shi(JP)

(72) Inventor: Okano, Kanemichi
2-3-610, Minase-2-chome Shimamotocho
Mishima-gun Osaka(JP)

(72) Inventor: Akira, Toshiaki
19-33, Yamatedai-6-chome
Ibaraki-shi(JP)

(74) Representative: Vossius Vossius Tauchner Heunemann
Rauh
Siebertstrasse 4 P.O. Box 86 07 67
D-8000 München 86(DE)

(54) Preventive and curative agent for ulcers of the gastro-intestinal tract.

(57) Described is a preventive and curative agent for ulcers of the gastro-intestinal tract comprising as the main constituent an active component having anti-ulcer activity obtained by extracting cassia buds with hot water, an alcohol or a water-alcohol mixture. This agent may be used for preparing pharmaceutical compositions suitable for oral or parenteral administration.

EP 0 142 036 A1

VOSSIUS · VOSSIUS · TAUCHNER · HEUNEMANN · RAUH

PATENTANWÄLTE

0142036

SIEBERTSTRASSE 4 · 8000 MÜNCHEN 86 · PHONE: (089) 47 40 75
CABLE: BENZOLPATENT MÜNCHEN · TELEX 5-29 453 VOPAT D

─1─

Our Ref.: T 315 EP

11. Okt. 1984

Case: A 8769-04

Priority: November 14, 1983
No. 213686/83, Japan

The Green Cross Corporation

Osaka, Japan


## Preventive and curative agent for ulcers of the gastro-intestinal tract


This invention relates to a preventive and curative agent
for ulcers of the gastro-intestinal tract comprising as
the main constituent an active component having anti-ulcer
activity derived from cassia buds. Cassia buds are immature fruits of Cinnamomum cassia Blume belonging to
Lauraceae.

The present inventors have investigated the pharmalogical
effects of a water-soluble component contained in cassia
bark. They have found that this component has strong
inhibitory activity on gastric acid secretion, improving
activity on gastric mucosal blood flow, accelerating
activity on gastric mucus secretion and accelerating
activity on gastric mucosa restoration. This component
has a similar inhibitory activity on gastric acid secretion as Cimetidine which, for the time being, is considered to be one of the most potent anti-ulcer agents having a preventive and curative effect also on stress ulcers and serotonin-induced ulcers of digestive organs.

The active component of this invention was obtained by extracting cassia buds with hot water, an alcohol or water-alcohol mixture, and it exhibits substantially the same activities as the above-mentioned water-soluble component of cassia bark. It was found, however, to give a spectrum of action somewhat different from that of the water-soluble component of cassia bark with respect to anti-ulcer activity on various ulcer models with experimental animals. Compared with the water-soluble component of cassia bark, the hot-water extract of cassia buds had a very low inhibitory activity on gastric ulcer induced by exposure to cold and restraint stress, it had substantially equal inhibitory effect on cysteamine duodenal ulcer, and showed a 54.3 % inhibition of serotonin-induced ulcer due to ischemic lesions of gastric mucosa compared to the degree of inhibition in the case of a control group when intraperitoneally administered at the dose of 20 mg/kg. This value indicates that the inhibitory activity of the hot-water extract of cassia buds is 3 times as high as that of the component of cassia bark.

Moreover, the hot-water extract of cassia buds has reduced toxicity. The $LD_{50}$ is 5,000 mg/kg or more when intraperitoneally administered to mice, and hence it was concluded to be very safe.

From the results described above, it can be taken that the active component having anti-ulcer activity derived from cassia buds improves and increases the gastric and duodenal mucosal blood flow to potentiate the preventing abilities and healing powers of the digestive organs against ulcer, and thus has excellent effects in preventing and healing gastric and duodenal ulcers.

It is an object of this invention to provide a novel preventive and curative agent for lesions of the mucosa and ulcers in the gastro-intestinal tract.

This invention provides a novel preventive and curative agent for lesions of the mucosa and ulcers of the gastro-intestinal tract comprising as the main constituent an active component having anti-ulcer activity obtained by extracting cassia buds with hot water, an alcohol or water-alcohol mixture (hereinafter referred to as "component A").

Cassia buds used as raw material are dark-brown immature fruits having a diameter of 3 to 5 mm, taste slightly sweet and bitter, and have a cinnamic odor weaker than that of cassia bark. In Kaneyoshi Akamatsu "Japanese and Chinese Medicines" Ishiyaku Publication Co, Ltd., it is described that cassia buds are effective in normalizing the functions of the liver in disease condition; in treating the stomach which has a reduced ability of digestion caused by cold stress or by too much fatigue according to the method "Warming" (a method for relieving symptoms of a failure of gastro-intestinal functions caused by a cold by using agents for warming the stomach and intestines); in assisting to the normalization of the renal functions and restoring disorders of the digestive functions; relieving from symptoms of vomitting; relieving from pain; and increasing the appetite. Cassia buds are also effective, for example, against stomach ache in the pit of the stomach caused by intemperate eating or drinking or by mental stimulus, and against pain accompanied by psychroesthesia due to the reduced ability of digestion of the stomach caused by cold stress or by overfatigue.

One concrete example for a process for obtaining "component A" is described in the following.

To 1 kg of dried cassia buds were added 10 liters of
water. The extraction was conducted by heating the
mixture to 50°C to 100°C for 1 to 10 hours. Preferably,
the mixture is gently boiled for 1 hour. Thereafter the
buds were removed by filtration to obtain an extract.
Usually, this procedure is repeated twice, and the two
extracts thus obtained are combined. In the case of extraction with an alcohol, the alcohol is used in an amount
10 times as high as that of cassia buds. The extraction
is carried out at room temperature or at a lower temperature
for 24 to 48 hours to obtain an alcohol extract. The
aqueous or alcoholic extract is evaporated under reduced
pressure, the residue is redissolved in water and the
aqueous solution is freeze-dried. The alcohol used in
the above-mentioned extraction is preferably methanol
or ethanol. Usually, the freeze-dried extract is obtained
in an amount of 5 to 10 % of the weight of dried cassia
buds. This extract is stable on storage in a refrigerator
at 2° to 10°C and is soluble in water and physiological
saline. Depending on the concentration, a small amount
of an insoluble material remains, but there is no adverse effect on the efficacy of the extract even when
the insoluble material is removed by filtration.

Pharmaceutical compositions, such as tablets or capsules,
may be prepared from the dried extract by adding there-
to an appropriate excipient. It is also possible to
prepare from dried extract an injection preparation by
dissolving it in a physiological isotonic solution,
for example, physiological saline.

Furthermore, purified "component A" is obtained by purifying the extract obtained in the manner described above
by using an appropriate method, for example, adsorption and recovery by using silica gel or ion exchange resins,
such as Amberlite®, butanol-water countercurrent fractionation, or the like. Like said extract, pharmaceutical
compositions for oral administration can be prepared
from "component A" in the form of e.g. tablets or
capsules, and also in the form of an injection preparation
by dissolving it in physiological saline, or in the form
of a solution prepared by adding thereto a small amount
of a dissolution adjuvant, or in the form of a dry injection preparation which is dissolved at the time of
use.

The extract of cassia buds and the "component A" of this
invention (both obtained in the Production Examples
described hereinafter) were tested for pharmacological
effects according to the following methods.

1. Serotonin ulcer

Serotonin-creatinine sulfate, at a dose of 30 mg/kg,
was subcutaneously administered to the dorsal part
of male Wistar rats weighing 160 to 180 g fasted
for 48 hours prior to the experiment. After 4 to 24
hours, the rats were subjected to laparotomy for evaluating
the drug potency. The ulcer index was expressed in
terms of the sum of the areas of hemorrhagic erosions.
Each test sample (the hot-water extract, "component A",
or physiological saline as control) was intraperitoneally
administered 30 minutes before the administration of
serotonin.

2. Ulcer induced by cold stress and restraint

Male SD strain rats weighing 160 to 180 g fasted for
24 hours were restrained in a wire net cage, left as
they were in a cold room at 4 $\pm$ 1°C for 5 hours, and then subjected to laparotomy followed by assay.
The ulcer index was expressed in terms of the sum
of the major areas of hemorrhagic erosions. Each
test sample was intraperitoneally administered 30
minutes before the beginning of the restraint.

3. Cysteamine-induced duodenal ulcer

Cysteamine hydrochloride was administered to female
Wistar rats (body wt. 250 to 300 g) fasted for 24 hours
under the skin of the back in an amount of 40 mg/kg.
18 hours later the rats were subjected to laparotomy,
and the areas of erosions formed in the duodenal
portion were measured. Each test sample was intraperitoneally (i.p.) administered 30 minutes before
the administration of cysteamine hydrochloride.

The test results of above 1., 2., and 3. are shown
in Table 1, Table 2 and Table 3, respectively.

Table 1

Inhibition test of Serotonin ulcer

| Test sample | 84% Inhibition dose ($ID_{84}$) |
|---|---|
| Hot-water extract of cassia buds | 41.0    mg/kg I.P. |
| "component A" | 0.03   mg/kg I.P. |
| Hot-water extract of cassia bark | 162.5    mg/kg I.P. |

Table 2

Inhibition test of ulcer induced by cold stress
and restraint

| Test sample | 84% Inhibition dose ($ID_{84}$) |
|---|---|
| Hot-water extract of cassia buds | Ineffective |
| Hot-water extract of cassia bark | 84.4   mg/kg I.P. |

Table 3

Inhibition test of cysteamine-induced duodenal ulcer

| Test sample | 84% Inhibition dose ($ID_{84}$) |
|---|---|
| Hot-water extract of cassia buds | 34.9   mg/kg I.P. |
| Hot-water extract of cassia bark | 34.7   mg/kg I.P. |

As shown above, the hot-water extract of cassia buds
and "component A" exhibited  a very high inhibitory
effect on serotonin ulcer. This indicates that this
substance and the "component A" maintain the microcircu-
lation of blood in the gastric and duodenal mucosa
and improve the resistance to ulceration. Thus they
are very useful and effective as medicines for the prevention and remedy of gastric and duodenal ulcers.

Although the effective dose of the hot-water extract or "component A" varies depending on symptoms to be cured or prevented, the effective dose of the extract is usually more than 10 mg/kg/day, preferably 40 to 50 mg/kg/day, and that of "component A" is usually more than 0.01 mg/kg/day, preferably 0.05 to 0.1 mg/kg/day.

This invention is further explained in more detail in the Production Examples and Examples, which should by no way limit but rather illustrate the invention.

Production Example 1

50 kg of cassia buds were added 100 liters of water, and extraction was conducted with heating at 100°C for 1 hour. This extraction procedure was repeated twice to obtain 200 liters of an aqueous extract. This extract was concentrated under reduced pressure to a volume of about 20 liters. The concentrated extract (concentrate) was used as the hot-water extract in the experimental example. The concentrate was centrifuged (7,000 r.p.m., 10 min). The supernatant was recovered from the sediment, and 12,000 cm$^3$ of Amberlite Ⓡ XAD-2 (manufactured by ORUGANO K.K.) was added to adsorb the "component A". After rinsing, the "component A" was eluted with 80 % aqueous methanol. The eluate was concentrated under reduced pressure, and 420 g of silica gel (manufactured by Merck & Co., Inc.) were added to the residue to adsorb the "component A".

The silica gel containing the "component A" was placed on a column packed with 6 kg of silica gel and eluted with 45 liters of methanol. The eluate was evaporated to dryness under reduced pressure. Then, 2 liters of water were added to dissolve the dried residue, and the resulting solution was extracted 5 times with butanol. The butanol extracts were combined. The butanol was removed by distillation under reduced pressure, and the residue was dissolved in 1 liter of water. The resulting solution was introduced into an Amberlite ® XAD-2 column and eluted with a 80 % aqueous methanol solution to obtain 10 liters of an eluate.

The eluate was evaporated to dryness under reduced pressure and then dissolved in 20 ml of 50 % aqueous methanol. The resulting solution was contacted with Toyopearl ® HW-40S (manufactured by TOYOSODA K.K.) to adsorb the "component A", which was then eluted with 50 % aqueous methanol. This product was used as the "component A" in the experimental example.

Production Example 2

10 liters of ethanol were added to 1 kg of cassia buds, and the resulting mixture was gently stirred at 60°C for 3 hours to obtain an ethanol extract. The extract was evaporated under reduced pressure and then redissolved in water, and the resulting solution was freeze-dried to obtain 100 g of a dried extract.

Production Example 3

10 liters of 50 % aqueous ethanol were added to 1 kg of cassia buds. The mixutre was maintained at room temperature for 24 to 48 hours to obtain an aqueous alcoholic extract. The extract was evaporated to dryness under reduced pressure and then redissolved
in water. The resulting solution was freeze-dried
to obtain 95 g of a dried extract.


Example 1

   Preparation of tablets

   (1) Hot water extract of cassia buds      100   mg

   (2) Fine grains No. 209 for direct
       tableting (mfd. by FUJI KAGAKU K.K.)   46.6 mg

           metasilicic acid
              magnesium aluminate       20%

           corn starch                  30%

           lactose                      50%

   (3) Crystalline celluose                      24.0 mg

   (4) Carboxymethyl cellulose·calcium salt   4.0 mg

   (5) Magnesium stearate                        0.4 mg


The components (1), (3) and (4) were previously passed
through a 100-mesh screen. Components (1), (3), (4) and
(2) were individually dried to a definite water content
and were then mixed in the above-mentioned weight proportion by means of a mixer. Subsequently, (5) was
added to the entirely homogeneous mixed powder thus
obtained and mixed therewith for a short period of time
(30 minutes). The resulting mixed powder was
tabletted (pounder: 6.3 mm , 6.0 mm R) to obtain
tablets. If necessary, the tablets may be coated with
conventional film coating agents soluble in the stomach
(e.g., polyvinyl acetal diethyaminoacetate) or edible
coloring agents.

- 11 -          0142036

Example 2

Tablets were prepared in the same manner as in
Example 1, except that (1) in Example 1 was
replaced by 100 mg of a methanol extract of cassia
buds or by the "component A" in an amount of 0.1
to 100 mg depending on its efficacy.

Example 3

Preparation of Capsules

| | | |
|---|---|---|
| (1) 50% Aqueous methanol extract of cassia buds | 50 g | |
| (2) Lactose | 935 g | |
| (3) Magnesium stearate | 15 g | |

The above-mentioned components (1), (2) and (3) were
individually weighed out and 1,000 g, in all, of
these were homogeneously mixed. The resulting mixed
powder was filled into hard gelatine capsules in
an amount of 200 mg each to prepare capsules for
oral administration.

Example 4

Capsules were prepared in the same manner as in
Example 3, except that (1) in Example 3 was replaced by 50 g of a methanol extract of cassia
buds or by the "component A" in an amount of 50
to 10,000 mg depending on its efficacy.

Example 5

Injection preparation

A vial or ampoule containing dried "component A" was prepared."Component A" was immediately dissolved in distilled water or physiological saline,
and used as an injection preparation.

Example 6

Injection preparation

An injection preparation according to the Japanese
Pharmacopoeia was prepared by adding the "component
A" alone or together with an appropriate stabilizer
and isotonicity, to an aqueous solution, and filling
the solution into ampoules.

Claims

1. A preventive and curative agent for ulcers of
   the gastro-intestinal tract comprising as the
   main constituent an active component having anti-
   ulcer activity obtained by extracting cassia buds
   with hot water, an alcohol or a water-alcohol
   mixture.

2. A process for producing a preventive and curative
   agent for ulcers of the gastro-intestinal tract,
   which comprises extracting cassia buds with hot
   water, an alcohol or a water-alcohol mixture and
   recovering the extract containing the active component having anti-ulcer activity.

3. Pharmaceutical composition containing the preventive and curative agent for ulcers of the gastro-
   intestinal tract according to claim 1 and a pharmaceutically acceptable carrier or diluent.

4. Pharmaceutical composition according to claim 3,
   in the form of a powder, a tablet or capsule for
   oral administration.

5. Pharmaceutical composition according to claim 3,
   in the form of a liquid for intravenous, intra-
   muscular or oral administration.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS, vol. 74, 1982, no. 55971; YAMAZAKI, KATSUHIRO et al.: "Inhibitory effects of stomachic crude drugs on digestive enzymes" & CHEM PHARM BULL (TOKYO) 29(10): 2966-2973. 1981 * Abstract * | 1-5 | A 61 K 35/78 |
| | --- | | |
| X | CHEMICAL ABSTRACTS, vol. 95, no. 5, August 3, 1981, page 47, no. 35420a, Columbus, Ohio, US; YAGI, AKIRA et al.: "Chemical studies on aqueous extract of cinnamoni cortex and its therapeutic trial for experimental nephritis" & WAKANYAKU SHINPOJUMU, (KIROKU) 1980, 13, 72-8 * Abstract * | 1-5 | |
| | --- | | **TECHNICAL FIELDS SEARCHED (Int. Cl.4)** |
| Y | CHEMICAL ABSTRACTS, vol. 96, no. 19, May 10, 1982, page 1, no. 154782a, Columbus, Ohio, US; NISHIOKA, ITSUO: "Constituents of cassia bark" & GENDAI TOYO IGAKU 1982, 3(1), 36-42 * Abstract * | 1-5 | A 61 K |
| | ---     -/- | | |

The present search report has been drawn up for all claims

| Place of search | Date of completion of the search | Examiner |
|---|---|---|
| THE HAGUE | 15-11-1984 | REMPP G.L.E. |

0142036

. Application number

European Patent
Office

EUROPEAN SEARCH REPORT

EP 84 11 2250

Page   2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOLOGICAL ABSTRACTS, vol. 73, 1982, no. 77896; NOHARA, TOSHIHIRO et al.: "Constituents of cinnamomi cortex: 5. Structures of 5 novel diterpenes, cinncassiol D1, cinncassiol D1 glucoside, cinncassiol D2, cinncassiol D2 glucoside and cinncassiol D3"& CHEM PHARM BULL (TOKYO) 29(9): 2451-2459. 1981 * Abstract * | 1-5 | |
| | --- | | |
| Y | BIOLOGICAL ABSTRACTS, vol. 73, 1982, no. 77897; KASHIWADA, YOSHIKI et al.: "Constituents of cinnamomi cortex: 4. Structures of cinncassiol C1 glucoside, cinncassiol C2 and cinncassiol C3" & CHEM PHARM BULL (TOKYO) 29(9): 2686-2688. 1981 * Abstract * | 1-5 | TECHNICAL FIELDS SEARCHED (Int. Cl.4) |
| | --- | | |
| Y | BIOLOGICAL ABSTRACTS, vol. 71, 1981, no. 47745; NOHARA, TOSHIHIRO et al.: "The constituents of cinnamomi cortex: 3. Structures of cinnassiol B and its glucoside" & CHEM PHARM BULL (TOKYO) 28(9): 2682-2686. 1980 * Abstract * | 1-5 | |
| | ---                    -/- | | |

The present search report has been drawn up for all claims

| Place of search THE HAGUE | Date of completion of the search 15-11-1984 | Examiner REMPP G.L.E. |
|---|---|---|

| | DOCUMENTS CONSIDERED TO BE RELEVANT | | Page 3 |
|---|---|---|---|
| **Category** | Citation of document with indication, where appropriate, of relevant passages | Relevant to claim | CLASSIFICATION OF THE APPLICATION (Int. Cl.4) |
| A | BIOLOGICAL ABSTRACTS, vol. 63, 1977, no. 19160; EGAWA, HIROSHI et al.: "The antimicrobial components found in the crude drugs" & BULL FAC AGRIC SHIMANE UNIV 9. 52-57. 1975(recd. 1976) ----- | | |
| | | | **TECHNICAL FIELDS SEARCHED (Int. Cl.4)** |

The present search report has been drawn up for all claims

| Place of search THE HAGUE | Date of completion of the search 15-11-1984 | Examiner REMPP G.L.E. |
|---|---|---|